# EUROPEAN PATENT APPLICATION

(11) **EP 4 603 953 A1**
(43) Date of publication of application: **20.08.2025**
(21) Application number: 23888897.8
(22) Date of filing: 14.09.2023
(51) Int. Cl.: G06F 3/01, G06F 3/0484, G06F 1/16, G06F 3/0481, A61B 5/024, A61B 5/318, A61B 5/053, G06F 3/04845, G06F 3/04842

(54) **ELECTRONIC DEVICE AND METHOD FOR CHANGING SHAPE OF VIRTUAL OBJECT**

(30) Priority: 11.11.2022 KR 20220150268; 22.11.2022 KR 20220157662
(71) Applicant: Samsung Electronics Co., Ltd., Suwon-si, Gyeonggi-do 16677 (KR)
(72) Inventor: IM, Byungjai, Suwon-si Gyeonggi-do 16677 (KR); KOO, Jihoon, Suwon-si Gyeonggi-do 16677 (KR); KIM, Jinsung, Suwon-si Gyeonggi-do 16677 (KR); AHN, Jaejin, Suwon-si Gyeonggi-do 16677 (KR); YOON, Sangil, Suwon-si Gyeonggi-do 16677 (KR); LEE, Seungho, Suwon-si Gyeonggi-do 16677 (KR); LEE, Taeyang, Suwon-si Gyeonggi-do 16677 (KR); JO, Yeonho, Suwon-si Gyeonggi-do 16677 (KR)
(74) Representative: Gulde & Partner
(86) International application number: PCT/KR2023/013822
(87) International publication number: WO 2024/101632

(57) **Abstract**

This wearable device may comprise a sensor, a display, and a processor. The processor may be configured to: by using a first software application, obtain data about a motion of the wearable device; on the basis of the data, identify a type of exercise performed by a user wearing the wearable device while the data was obtained; by using a second software application, identify whether the type of exercise corresponds to a type of virtual animal character; in response to a type of exercise that is different from the type of virtual animal character, change a stored first value to a second value; identify a shape of a virtual object on the basis of the second value; in response to the type of exercise corresponding to the type of virtual animal character, change the first value to a third value that is higher than the second value; identify the shape of the virtual object on the basis of the third value; and display the virtual object having the identified shape. The second value and the third value may be identified on the basis of information about the duration of exercise and information about calories burned due to exercise.

## Description

### [Technical Field]

The following descriptions relate to an electronic device and a method for changing a shape of a virtual object. Specifically, the following descriptions relate to the electronic device and the method for obtaining a value for changing the shape of the virtual object based on a result of an exercise of a user.

### [Background Art]

A wearable device may be worn by a user. For example, the wearable device may be worn on a wrist, a head, and other parts of the user. For example, the wearable device may include a device in a form of a watch, a device in a form of glasses, and the like. The wearable device may obtain data for a motion of the user. For example, the wearable device may obtain the data for the motion via a sensor. The wearable device may display a virtual object via a display. For example, the wearable device may display the virtual object by using a software application.

### [Disclosure]

### [Technical Solution]

A wearable device is provided. The wearable device may comprise a sensor. The wearable device may comprise a display. The wearable device may comprise a processor. The processor may be configured to obtain data for a motion of the wearable device via the sensor by using a first software application for health. The processor may be configured to, based on the data, identify a type of an exercise performed by a user wearing the wearable device while the data is obtained by using the first software application. The processor may be configured to identify whether the type of the exercise corresponds to a type of a virtual animal character by using a second software application for displaying a visual object for a virtual animal character. The processor may be configured to, in response to the type of the exercise being different from the type of the virtual animal character, change a first value stored for changing a shape of the virtual object to a second value higher than the first value and identified based on the data, and identify a shape of the virtual object based on the second value. The processor may be configured to, in response to the type of the exercise being corresponding to the type of the virtual animal character, change the first value to a third value higher than the second value and identified based on the data, and identify a shape of the virtual object based on the third value. The processor may be configured to, in response to a predetermined event, display via the display the visual object having the identified shape. The second value and the third value may be identified based on information on exercising time of the exercise and information on consumption calories according to the exercise identified from the data.

A method performed by a wearable device is provided. The method may comprise obtaining data for a motion of the wearable device via a sensor of the wearable device by using a first software application for health. The method may comprise, based on the data, identifying a type of an exercise performed by a user wearing the wearable device while the data is obtained by using the first software application. The method may comprise identifying whether the type of the exercise corresponds to a type of a virtual animal character by using a second software application for displaying a visual object for a virtual animal character. The method may comprise, in response to the type of the exercise being different from the type of the virtual animal character, changing a first value stored for changing a shape of the virtual object to a second value higher than the first value and identified based on the data, and identifying a shape of the virtual object based on the second value. The method may comprise, in response to the type of the exercise being corresponding to the type of the virtual animal character, changing the first value to a third value higher than the second value and identified based on the data, and identifying a shape of the virtual object based on the third value. The method may comprise, in response to a predetermined event, displaying via a display of the wearable device the visual object having the identified shape. The second value and the third value are identified based on information on exercising time of the exercise and information on consumption calories according to the exercise identified from the data.

### [Description of the Drawings]

FIG. 1 is a block diagram of an electronic device in a network environment according to various embodiments.
FIG. 2 illustrates an example of obtaining data for a motion of a user via a wearable device according to an embodiment of the present disclosure.
FIG. 3A illustrates an example of displaying a visual object for a virtual animal character via a wearable device according to an embodiment of the present disclosure.
FIG. 3B illustrates an example of a block diagram of a wearable device according to an embodiment of the present disclosure.
FIG. 4 is a flowchart illustrating an example of a method of identifying a shape of a visual object for a virtual animal character based on data for a motion according to an embodiment of the present disclosure.
FIGS. 5A and 5B illustrate examples of changing a shape of a visual object for a virtual animal character based on data for a motion according to an embodiment of the present disclosure.
FIG. 6A is a flowchart illustrating an example of a method of proposing a virtual animal character identified based on data for a motion according to an embodiment of the present disclosure.
FIG. 6B illustrates an example of proposing a virtual animal character identified based on data for a motion according to an embodiment of the present disclosure.
FIG. 7A is a flowchart illustrating an example of transmitting a value for changing a shape of a visual object for a virtual animal character to another wearable device according to an embodiment of the present disclosure.
FIG. 7B illustrates an example of changing a shape of a virtual object displayed via another wearable device based on a value for changing a shape of a visual object for a virtual animal character according to an embodiment of the present disclosure.
FIG. 7C illustrates another example of changing a shape of a virtual object displayed via another wearable device based on a value for changing a shape of a visual object for a virtual animal character according to an embodiment of the present disclosure.

### [Mode for Invention]

Terms used in the present disclosure are used only to describe a specific embodiment, and may not be intended to limit a range of another embodiment. A singular expression may include a plural expression unless the context clearly means otherwise. Terms used herein, including a technical or a scientific term, may have the same meaning as those generally understood by a person with ordinary skill in the art described in the present disclosure. Among the terms used in the present disclosure, terms defined in a general dictionary may be interpreted as identical or similar meaning to the contextual meaning of the relevant technology and are not interpreted as ideal or excessively formal meaning unless explicitly defined in the present disclosure. In some cases, even terms defined in the present disclosure may not be interpreted to exclude embodiments of the present disclosure.

In various embodiments of the present disclosure described below, a hardware approach will be described as an example. However, since the various embodiments of the present disclosure include technology that uses both hardware and software, the various embodiments of the present disclosure do not exclude a software-based approach.

A term referring to a configuration of a device (e.g., a processor, a camera, a display, a module, a pen, a communication circuit, and the like), a term for a calculation state (e.g., a step, an operation, and a procedure), a term referring to a signal (e.g., a signal, information, data, a stream, a user input, an input, and the like), and a term referring to data (e.g., a parameter, a value, and the like), used in the following description are exemplified for convenience of explanation. Therefore, the present disclosure is not limited to terms to be described below, and another term having an equivalent technical meaning may be used.

In addition, in the present disclosure, the term 'greater than' or 'less than' may be used to determine whether a particular condition is satisfied or fulfilled, but this is only a description to express an example and does not exclude description of 'greater than or equal to' or 'less than or equal to'. A condition described as 'greater than or equal to ' may be replaced with 'greater than', a condition described as 'less than or equal to' may be replaced with 'less than', and a condition described as ' greater than or equal to and less than' may be replaced with 'greater than and less than or equal to'. In addition, hereinafter, 'A' to 'B' refers to at least one of elements from A (including A) to B (including B).

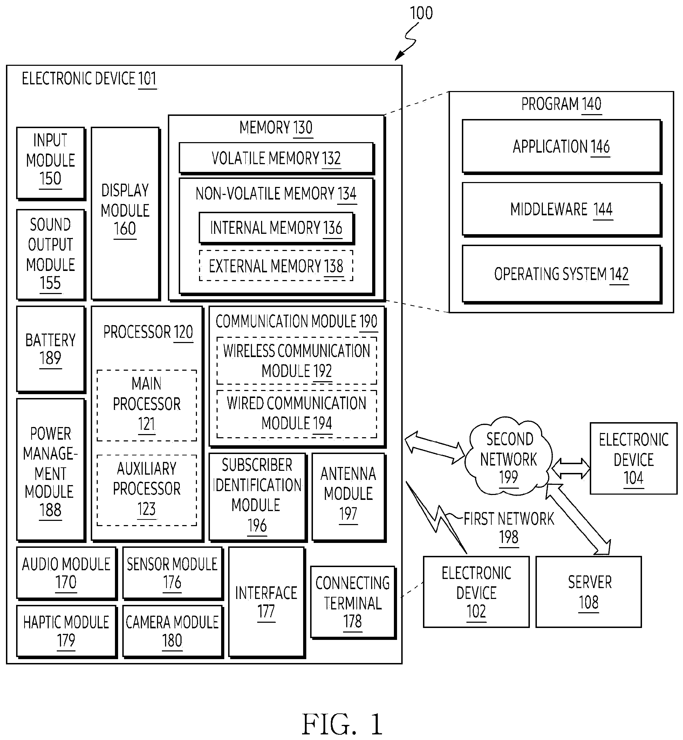

FIG. 1 is a block diagram of an electronic device in a network environment according to various embodiments.

Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

A following first software application may indicate a software application for health management (or health) of a user. In addition, a second software application may indicate a software application for displaying a virtual animal character. The virtual animal character may include a virtual animal character as well as an animal character that actually exists. For example, the virtual animal character may include a frog, a lion, and a chicken. For example, the virtual animal character may include an extinct animal or a virtual animal.

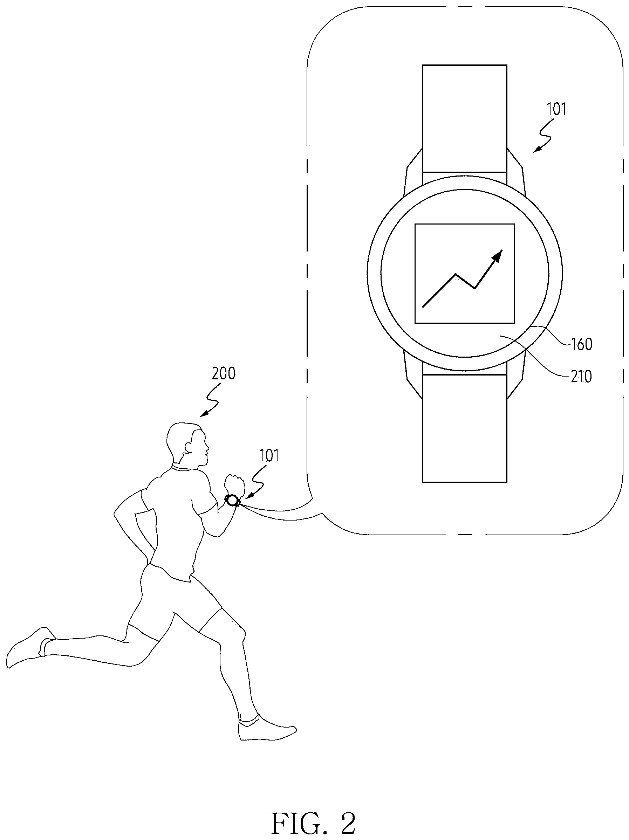

FIG. 2 illustrates an example of obtaining data for a motion of a user via a wearable device according to an embodiment of the present disclosure. The motion may include a linear exercise and a rotational exercise. For example, a change in the motion may include a movement of a representative point displaying a wearable device (or a user) and a change (i.e., a change in an orientation) in a reference direction related to the wearable device (or the user).

A wearable device 101 of FIG. 2 may be understood to be identical to the electronic device 101 of FIG. 1. For example, a display 160 included in the wearable device 101 of FIG. 2 may be understood to be substantially identical to the display module 160 of FIG. 1.

FIG. 2 illustrates an example in which a user 200 performs an exercise. For example, the user 200 may perform running. At this time, the user 200 may be in a state in which the wearable device 101 is worn. For example, the user 200 may wear the wearable device 101 in a form of a watch and perform an exercise in a state of wearing it.

The wearable device 101 may obtain data related to an exercise of the user 200. For example, the wearable device 101 may execute a first software application and obtain the data related to the exercise of the user 200 via a sensor (e.g., the sensor module 176 of FIG. 1) of the wearable device 101 by using the first software application. The first software application may be executed on a background or a foreground of the wearable device 101. The data related to the exercise may include data for a motion of the wearable device 101 that may be changed according to performance of the exercise of the user 200. The sensor may include at least one of a global positioning system (GPS), a gyro sensor, a geomagnetic sensor, a photoplethysmogram (PPG) sensor, an electrocardiogram (ECG) sensor, or a bioelectrical impedance analysis sensor.

The first software application may identify exercising time for the exercise performed by the user 200 based on the data for the motion. The first software application may identify consumption calories according to the exercise performed by the user 200 based on the data for the motion. The wearable device 101 may display a result of the exercise performed by the user 200. For example, the wearable device 101 may display the result of the exercise via the display 160 by using a user interface 210 of the first software application. For example, the result of the exercise may include information on the exercising time or information on the consumption calories.

As described above, the wearable device may provide information to the user by displaying the result of the exercise performed by the user by using a software application. The provided information may indicate a result (e.g., consumption calories, an exercising time, and the like) of an exercise simply processed based on data for a motion of the wearable device as the user performs an exercise. In case that only the simple result of the exercise is provided, the user may lack motivation for performing the exercise. Therefore, a method is required to provide a more specific user experience while simultaneously motivating the user to perform the exercise.

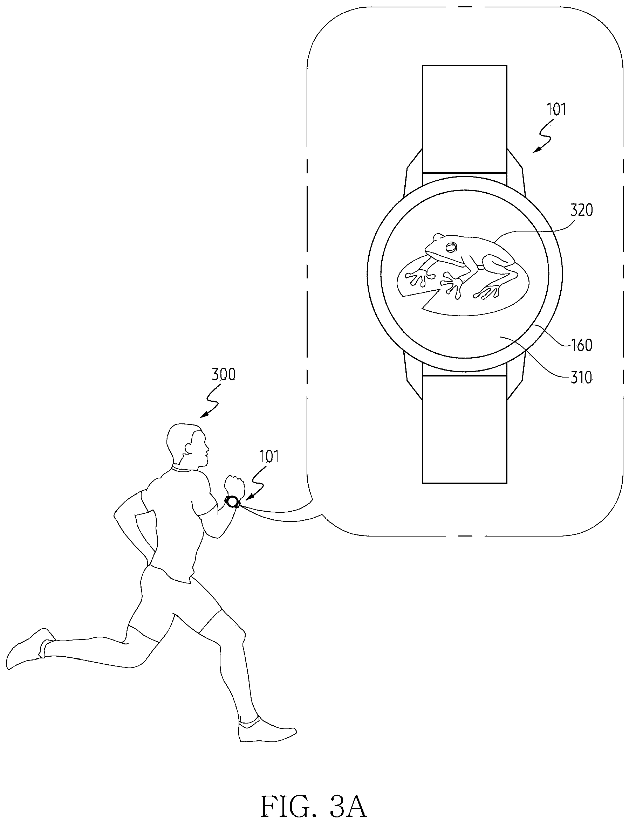

FIG. 3A illustrates an example of displaying a visual object for a virtual animal character via a wearable device according to an embodiment of the present disclosure.

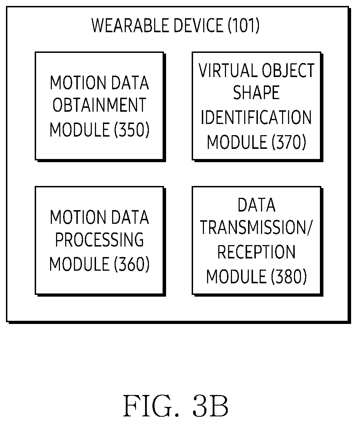

A wearable device 101 of FIG. 3A may be understood to be identical to the electronic device 101 of FIG. 1. For example, a display 160 included in the wearable device 101 of FIG. 3 may be understood to be substantially identical to the display module 160 of FIG. 1.

FIG. 3 illustrates an example in which a user 300 performs an exercise. For example, the user 300 may perform running. At this time, the user 300 may be in a state in which the wearable device 101 is worn. For example, the user 300 may wear the wearable device 101 in a form of a watch and perform an exercise in a state of wearing it.

The wearable device 101 may obtain data related to an exercise of the user 300. For example, the wearable device 101 may execute a first software application and obtain the data related to the exercise of the user 300 via a sensor (e.g., the sensor module 176 of FIG. 1) of the wearable device 101 by using the first software application. The first software application may be executed on a background or a foreground of the wearable device 101. The data related to the exercise may include data for a motion of the wearable device 101 that may be changed according to performance of the exercise of the user 300. The motion may include a linear exercise and a rotational exercise. For example, a change in the motion may include a movement of a representative point displaying a wearable device (or a user) and a change (i.e., a change in an orientation) in a reference direction related to the wearable device (or the user). The sensor may include at least one of a global positioning system (GPS), a gyro sensor, a geomagnetic sensor, a photoplethysmogram (PPG) sensor, an electrocardiogram (ECG) sensor, or a bioelectrical impedance analysis sensor.

The wearable device 101 may identify a type of an exercise performed by a user wearing the wearable device while the data for the motion is obtained by using the first software application.

The wearable device 101 may identify exercising time for the exercise performed by the user 300 based on the data for the motion by using the first software application. The wearable device 101 may identify consumption calories according to the exercise performed by the user 300 based on the data for the motion by using the first software application.

The wearable device 101 may display a result of the exercise performed by the user 300. For example, the wearable device 101 may display a visual object 320 for a virtual animal character via the display 160 by using a user interface 310 of a second software application. For example, the visual object 320 may be a frog. The visual object 320 may indicate an object for representing the virtual animal character.

The virtual animal character may be any animal character that may be selected in response to an input of the user 300. For example, the user 300 may execute a character selection function in response to an input to a user interface of the second software application of the wearable device 101. Accordingly, any animal character may be identified as the virtual animal character. Alternatively, any relevant virtual animal character may be identified as the virtual animal character based on information (e.g., a usual exercise habit of the user 300) stored in the first software application. In case that the user 300 usually performs an aquatic exercise such as swimming, the relevant any virtual animal character may indicate an aquatic organism character.

In FIG. 3A, an example in which the wearable device 101 displays the visual object 320 via the user interface 310 of the second software application is illustrated, but an embodiment of the present disclosure is not limited thereto. For example, the wearable device 101 may display only the visual object 320 via another user interface other than the second software application. In addition, for example, the wearable device 101 may display the visual object 320 corresponding to the virtual animal character via the first software application for health management other than using the second software application.

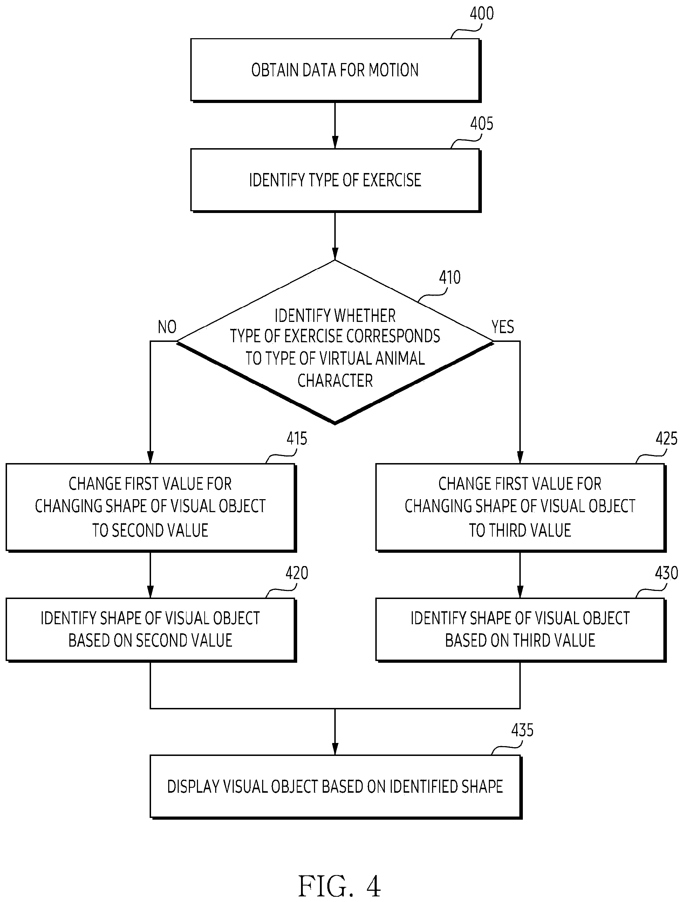

For example, a shape of the visual object 320 may be identified based on a result of the exercise performed by the user 300 and a type of the virtual animal character, and a type of an exercise performed by a user 200. The result of the exercise may include information on the exercising time and information on the consumption calories. Identifying the shape of the visual object 320 based on the type of the exercise and the type of the virtual animal character will be described in detail below in FIG. 4.

The type of the virtual animal character may include land, aqua, and air. In case that the virtual animal character is an animal that inhabits land, the type of the virtual animal character may be land. In case that the virtual animal character is an animal that that inhabits an underwater, the type of the virtual animal character may be aqua. In case that the virtual animal character is an animal that flies, the type of the virtual animal character may be air. The type of the virtual animal character may include at least one of land, aqua, and air. For example, in case that the virtual animal character is a lion, the type may include land. For example, in case that the virtual animal character is a frog, the type may include the land and the aqua. In case that the virtual animal character is a chicken, the type may include the land and the air.

The type of the exercise may be determined to correspond to the type of the virtual animal character. For example, the type of the exercise may include a land exercise, an aquatic exercise, and an aerial exercise. The land exercise may indicate a horizontal exercise performed on land. The aquatic exercise may indicate a horizontal exercise performed on an underwater, a surface of water, or ice. The aerial exercise may include a vertical movement, flight, or an exercise in a form of throwing an object and a mountain exercise.

As described above, a device and a method for obtaining a value for changing a shape of a virtual object (e.g., a visual object for a virtual animal character) according to embodiments of the present disclosure may motivate a user to perform an exercise. In addition, the device and the method for obtaining the value for changing the shape of the virtual object according to embodiments of the present disclosure may provide the user with a continuous user experience in which an exercise in a real environment is linked to a virtual environment by changing another virtual object in a virtual environment that may be provided via another wearable device.

FIG. 3B illustrates an example of a block diagram of a wearable device according to an embodiment of the present disclosure. The wearable device 101 of FIG. 3B may be understood to be substantially identical to the electronic device 101 of FIG. 1.

Referring to FIG. 3B, the wearable device 101 may include a plurality of components for changing a shape of a virtual object (e.g., a virtual animal character) based on data obtained while a user performs an exercise. For example, the wearable device 101 may include a motion data obtainment module 350, a motion data processing module 360, a virtual object shape identification module 370, and a data transmission/reception module 380. The motion data obtainment module 350, the motion data processing module 360, the virtual object shape identification module 370, and the data transmission/reception module 380 may be controlled or executed by a processor (e.g., the processor 120 of FIG. 1) of the wearable device 101.

In case that the user of the wearable device 101 performs an exercise, the motion data obtainment module 350 may obtain data for a motion of the wearable device 101 via a sensor. The sensor may include at least one of a global positioning system (GPS), a gyro sensor, a geomagnetic sensor, a photoplethysmogram (PPG) sensor, an electrocardiogram (ECG) sensor, and a bioelectrical impedance analysis sensor. For example, the wearable device 101 may obtain the data for the motion by using the first software application based on the motion data obtainment module 350.

The motion data processing module 360 may process data for the obtained motion in case that the user of the wearable device 101 performs an exercise. For example, the motion data processing module 360 may compute a value for changing a shape of a virtual object based on a result of the exercise identified based on the data for the motion. For example, the result of the exercise may include information on exercising time of the exercise performed by the user and information on consumption calories while the user performs the exercise. The information on the exercising time may include a first partial value for any unit time. The first partial value may indicate a parameter for a value for changing the shape of the virtual object. The information on the consumption calories may include a second partial value for any unit of consumption calories. The second partial value may indicate a parameter for a value for changing the shape of the virtual object. The value for changing the shape of the virtual object may be identified based on the first partial value, the second partial value, and a weight value identified based on the type of the exercise and a type of the virtual object.

The virtual object shape identification module 370 may identify the shape of the virtual object based on a value for identifying the shape of the visual object. For example, in case that the value for changing the shape of the virtual object is greater than or equal to a first reference value, the shape of the virtual object may be changed from a first shape to a second shape. For example, in case that the value for changing the shape of the virtual object is greater than or equal to the second reference value, the shape of the virtual object may be changed from the second shape to a third shape. The first shape to the third shape are merely an example for convenience of a description, and the shape of the visual object according to an embodiment of the present disclosure is not limited to including only three shapes. In addition, the virtual object shape identification module 370 may change a portion of the shape of the virtual object by identifying a partial type (e.g., an upper body type and a lower body type) for a type of an exercise. For example, in case that the partial type is the upper body type, the virtual object shape identification module 370 may change a portion (e.g., the upper body part) of the shape of the virtual object corresponding to the upper body type.

The data transmission/reception module 380 may indicate a module for transmitting/receiving data to/from another wearable device (not illustrated) connected to the wearable device 101. For example, the wearable device 101 may transmit a value for changing the shape of the virtual object to the other wearable device. The other wearable device may change a shape of another visual object in a virtual environment that may be displayed via the other wearable device based on the value for changing the shape of the virtual object.

FIG. 4 is a flowchart illustrating an example of a method of identifying a shape of a visual object for a virtual animal character based on data for a motion according to an embodiment of the present disclosure.

The motion may include a linear exercise and a rotational exercise. For example, the change in the motion may include a movement of a representative point displaying a wearable device (or a user) and a change (i.e., a change in an orientation) in a reference direction related to the wearable device (or the user). The visual object may indicate an object displayed via the wearable device to represent the virtual animal character. The shape may indicate a form of an exterior of the visual object.

The flowchart of FIG. 4 may be performed by the wearable device 101 of FIGS. 3A and 3B. For example, the flowchart of FIG. 4 may be performed by a processor (e.g., the processor 120 of FIG. 1) of the wearable device 101.

Referring to FIG. 4, in an operation 400, a processor may obtain data for a motion. For example, the processor may obtain data for a motion of the wearable device 101 via a sensor (e.g., the sensor module 176 of FIG. 1) by using a first software application for health. For example, the data for the motion may include data identified as the wearable device 101 moves or changes in a posture. Although not illustrated in FIG. 4, before performing the operation 400, the processor may execute the first software application. For example, the processor may execute the first software application in a background. Alternatively, the processor may execute the first software application in a foreground. The processor may obtain the data for the motion of the wearable device 101 via the sensor by using the first software application being executed.

For example, the sensor may include at least one of a global positioning system (GPS), a gyro sensor, a geomagnetic sensor, a photoplethysmogram (PPG) sensor, an electrocardiogram (ECG) sensor, or a bioelectrical impedance analysis sensor.

The data for the motion may be used to identify whether a user performs an exercise. For example, the processor may detect whether the user performs the exercise by identifying that the data for the motion is changed according to the exercise performance of the user.

In an operation 405, the processor may identify a type of an exercise. For example, the processor may identify the type of the exercise performed by the user by using the first software application based on the data for the motion. The processor may identify the exercise performed by the user wearing the wearable device 101 while data is obtained as the data for the motion is changed. The processor may identify a kind of the exercise performed by the user, based on the identified exercise. For example, the kind of the exercise may include various kinds of an exercise such as walking, running, biking, hiking, strength training, boxing, basketball, dancing, swimming, rowing machine, boating, water skiing, snorkeling, ice hockey, canoeing, water skiing, mountain biking, skiing, rock climbing, mountaineering, stair climbing, discus throwing, archery, or hang gliding. The kind of the exercise may be previously stored in memory (e.g., the memory 130 of FIG. 1) of the wearable device 101.

According to an embodiment, the type of the exercise may include a land exercise, an aquatic exercise, and an aerial exercise. The land exercise may indicate a horizontal exercise performed on land. The aquatic exercise may indicate a horizontal exercise or a vertical exercise performed on an underwater, a surface of water, or ice. The aerial exercise may include a vertical movement, flight, or an exercise in a form of throwing an object and a mountain exercise. For example, from among the above-described exercises, walking, running, biking, hiking, strength training, boxing, basketball, and dancing may be classified as a land exercise. In addition, from among the above-described exercises, swimming, rowing machine, boating, water skiing, snorkeling, ice hockey, canoeing, and diving may be classified as an aquatic exercise. In addition, from among the above-described exercises, mountain biking, skiing, rock climbing, mountaineering, stair climbing, discus throwing, archery, and hang gliding may be classified as an aerial exercise. Therefore, the processor may identify the exercise based on data for a motion related to the user, and may identify the type of the exercise in response to the identified exercise and the kinds of pre-stored exercise. However, the exercise may not be classified as just one type of an exercise. For example, as skiing is a vertical downhill exercise on snow, it may be an aquatic exercise and an aerial exercise.

According to an embodiment, the type of the exercise may be determined to correspond to a type of a virtual animal character displayed via a second software application. For example, the type of the virtual animal character may include land, aqua, and air, and correspondingly, the type of the exercise may include a land exercise, an aquatic exercise, or an aerial exercise.

According to an embodiment, the processor may identify the type of the exercise based on an input of the user. For example, based on an exercise or the type of the exercise inputted via a display of the wearable device 101 by using a user interface of the first software application, the processor may identify the type of the exercise.

In an operation 410, the processor may identify whether the type of the exercise corresponds to the type of the virtual animal character. For example, the processor may identify whether the type of the identified exercise corresponds to the type of the virtual animal character that may be displayed via the second software application. The second software application may indicate a software application for displaying a visual object for the virtual animal character. According to an embodiment, the processor may identify the type of the virtual animal character via the second software application. For example, the processor may identify the type of the virtual animal character stored in the second software application. Although not illustrated in FIG. 4, the processor may execute the second software application before the operation 410. The second software application may be executed in the background or the foreground.

In case that the type of the identified exercise does not correspond to the type of the virtual animal character, the processor may perform an operation 415. In case that the type of the identified exercise corresponds to the type of the virtual animal character, the processor may perform an operation 425. Meaning of the corresponding may indicate that the type of the identified exercise may be the same as the type of the virtual animal character.

In the operation 415, the processor may change a first value for changing the shape of the visual object to a second value. For example, in case that the type of the exercise does not correspond to the type of the virtual animal character, the processor may identify a first weight value. The first weight value may indicate a weight used to convert into a value for changing the shape of the visual object based on a first partial value and a second partial value for the exercise performed by the user. For example, it is assumed that the user performs 30 minutes of running, which is a land exercise, and consumes 20 kcal, and the first partial value is 1 point per 5 minutes of exercising time and the second partial value is 1 point per 10 kcal of consumption calories. At this time, in case that the virtual animal character is a shark, the first weight value may be set to 1. Accordingly, the processor may identify the second value that adds 8 (=(6+2)*1) points to the first value. In the example, the first partial value may be 6 points, and the second partial value may be 2 points. According to an embodiment, the second value may be stored by the processor, and the stored second value may be used as the first value thereafter. In case of being initially stored, the first value (i.e., an initial value) may be 0 point.

In an operation 420, the processor may identify the shape of the visual object based on the second value. For example, the processor may identify whether the second value exceeds a reference value. In case that the second value exceeds the reference value, the processor may set the shape of the visual object to a second shape changed from a first shape. In contrast, in case that the second value is less than or equal to the reference value, the processor may maintain the shape of the visual object as the first shape. For example, in case that a virtual animal character represented by the visual object is a frog, the first shape may be a tadpole and the second shape may be a frog. Alternatively, in case that a virtual animal character represented by the visual object is a dog, the first shape may be a puppy, and the second shape may be a dog. In other words, the change from the first shape to the second shape may indicate that the shape of the visual object is changed as a whole. In the above-described example, it is illustrated that the shape of the visual object includes two shapes, but an embodiment of the present disclosure is not limited thereto. For example, the shapes of the visual object may include four shapes, and accordingly, the reference value may include a first reference value, a second reference value, and a third reference value. The first reference value may be set lower than the second reference value, and the second reference value may be set lower than the third reference value.

According to an embodiment, the processor may identify a partial type for a type of an exercise. The partial type may be related to a body part of the user in which the exercise is performed. For example, the partial type may include an upper body type and a lower body type. Alternatively, the partial type may include an arm type, a chest type, a shoulder type, a back type, a thigh type, a calf type, and an ankle type. According to an embodiment, the processor may change a shape of a visual object based on the identified partial type. For example, in case that the second value is less than the reference value and the partial type identified corresponding to the second value is the upper body type, the processor may change the shape of the visual object from the first shape to a third shape. Herein, the third shape may be a shape in which a portion (e.g., an upper body part) of the first shape corresponding to the partial type is changed. In other words, the change from the first shape to the third shape may indicate that the shape of the visual object is partially changed.

In the operation 425, the processor may change the first value for changing the shape of the visual object to a third value. The third value may be a value greater than the second value identified in the operation 415. For example, when the type of the exercise corresponds to the type of the virtual animal character, the processor may identify a second weight value. The second weight value may indicate a weight used to convert into a value for changing the shape based on the first partial value and the second partial value for an exercise performed by the user. For example, it is assumed that the user performs 30 minutes of running, which is a land exercise, and consumes 20 kcal, and the first partial value is 1 point per 5 minutes of exercising time and the second partial value is 1 point per 10 kcal of consumption calories. At this time, in case that the virtual animal character is a lion, the first weight value may be set to 2. Accordingly, the processor may identify the third value that adds 16 (=(6+2)*2) points to the first value. The third value may be stored by the processor, and the stored third value may be used as the first value thereafter. In case of being initially stored, the first value (i.e., the initial value) may be 0 point.

In an operation 430, the processor may identify the shape of the visual object based on the third value. For example, the processor may identify whether the third value exceeds the reference value. In case that the third value exceeds the reference value, the processor may set the shape of the visual object to the second shape changed from the first shape. In contrast, in case that the third value is less than or equal to the reference value, the processor may maintain the shape of the visual object as the first shape. For example, in case that a virtual animal character represented by the visual object is a frog, the first shape may be a tadpole and the second shape may be a frog. Alternatively, in case that the virtual animal character represented by the visual object is a dog, the first shape may be a puppy, and the second shape may be a dog. In other words, the change from the first shape to the second shape may indicate that the shape of the visual object is changed as a whole. In the above-described example, it is illustrated that the shape of the visual object includes two shapes, but an embodiment of the present disclosure is not limited thereto. For example, the shapes of the visual object may include four shapes, and accordingly, the reference value may include the first reference value, the second reference value, and the third reference value. The first reference value may be set lower than the second reference value, and the second reference value may be set lower than the third reference value. According to an embodiment, the processor may display a remaining value from a value (e.g., the second value or the third value) for changing the shape of the visual object to one reference value of the reference values together with the visual object. The one reference value may indicate the closest reference value to a value for changing the shape of the current visual object.

According to an embodiment, the processor may identify a partial type for a type of an exercise. The partial type may be related to the body part of the user in which the exercise is performed. For example, the partial type may include an upper body type and a lower body type. Alternatively, the partial type may include an arm type, a chest type, a shoulder type, a back type, a thigh type, a calf type, and an ankle type. According to an embodiment, the processor may change the shape of the visual object based on the identified partial type. For example, in case that the third value is less than the reference value and the partial type identified corresponding to the third value is the upper body type, the processor may change the shape of the visual object from the first shape to the third shape. Herein, the third shape may be a shape in which a portion (e.g., an upper body part) of the first shape corresponding to the partial type is changed. In other words, the change from the first shape to the third shape may indicate that the shape of the visual object is partially changed.

In an operation 435, the processor may display a visual object based on the identified shape. For example, the processor may display the visual object having the identified shape via the display of the wearable device 101 in response to a predetermined event. The predetermined event may include an execution of the second software application or a response to a predefined user input (e.g., three touch inputs to the display, and the like) and the like. For example, the processor may display a visual object based on the first shape identified via the operation 420 or the operation 430. Alternatively, the processor may display a visual object based on the second shape identified via the operation 420 or the operation 430. Alternatively, the processor may display a visual object based on the third shape identified via the operation 420 or the operation 430.

For example, the processor may display a virtual animal character identified based on an input of the user via the display (e.g., the display module 160 of FIG. 1). For example, a visual object representing the identified virtual animal character may be displayed by using the user interface of the second software application. Alternatively, the visual object representing the identified virtual animal character may be displayed via a user interface of another software application based on information on the visual object in a state that the second software application is executed in a background. Alternatively, the visual object representing the identified virtual animal character may be displayed on a home screen of the wearable device in the state that the second software application is executed in the background.

According to an embodiment, the visual object representing the identified virtual animal character may be shared with the user of the wearable device and other users. For example, the information on the visual object may be provided to users of other accounts stored in an account of the user. In addition, the visual object may be uploaded to SNS of the user and provided to other users.

According to an embodiment, the processor may display information on a result of an exercise (e.g., exercising time, an exercise type, and consumption calories by an exercise) together with the visual object.

Referring to FIG. 4, a case in which each of a type of an exercise and a type of a virtual animal character is two and a case in which a weight thereof is two are described as an example, but an embodiment of the present disclosure is not limited thereto. As described above, an embodiment of the present disclosure may include a case in which the type of the exercise and the type of the virtual animal characters are three. A value of weights corresponding to the case in which the type of the exercise and the type of the virtual animal character are three may be set as illustrated in the following table.

**[Table 1]**

| | Land exercise | Aquatic exercise | Aerial exercise |
|---|---|---|---|
| Land organism | 2 | 1.2 | 1 |
| Aquatic organism | 1.2 | 2 | 1 |
| Aerial organism | 1.2 | 1 | 2 |

Referring to the above-described table, in case that a type of a virtual animal character is the land organism , a weight value may be set to 2 in case of the land exercise, a weight value may be set to 1.2 in case of the aquatic exercise, and, a weight value may be set to 1 in case of the aerial exercise. In case that a type of a virtual animal character is the aquatic organism, a weight value may be set to 1.2 in case of the land exercise, a weight value may be set to 2 in case of the aquatic exercise, and a weight value may be set to 1 in case of the aerial exercise. In case that a type of a virtual animal character is the aerial organism, and a weight value may be set to 1.2 in case of the land exercise, a weight value may be set to 1 in case of the aquatic exercise, and a weight value may be set to 2 in case of the aerial exercise.

In addition, referring to FIG. 4, a case in which a type of an exercise to be identified is one of a plurality of types is described as an example, but an embodiment of the present disclosure is not limited thereto. For example, the type of the identified exercise may be identified as including the plurality of types. For example, in case that the user is diving, a type of an exercise is an aquatic exercise and a vertical exercise, which may include an aerial exercise. That is, the type of the exercise for diving may be the aquatic exercise and the aerial exercise. In this case, a value of weights according to the type of the virtual animal character may be set as illustrated in the following table.

**[Table 2]**

| | Aquatic exercise | Aerial exercise |
|---|---|---|
| Land organism | 1.2 | 1 |
| Aquatic organism | 2 | 1 |
| Aerial organism | 1 | 2 |

Referring to the above-described table, in case that a type of a virtual animal character is the land organism, a weight value may be set to 1.2 in case of the aquatic exercise, and a weight value may be set to 1 in case of the aerial exercise. In case that a type of a virtual animal character is the aquatic organism, a weight value may be set to 2 in case of the aquatic exercise, and a weight value may be set to 1 in case of the aerial exercise. In case that a type of a virtual animal character is the aerial organism, a weight value may be set to 1 in case of the aquatic exercise, and a weight value may be set to 2 in case of the aerial exercise. According to an embodiment, a value for changing a shape of a visual object may be identified based on a maximum value from among the weight values. For example, in case that the virtual animal character is the land organism, a value for changing a shape of the visual object according to a result of diving may be identified based on the weight value of 1.2. For example, in case that a virtual animal character is the aquatic organism, the value for changing the shape of the visual object according to the result of diving may be identified based on the weight value of 2. For example, in case that a virtual animal character is the aerial organism, the value for changing the shape of the visual object according to the result of diving may be identified based on the weight value of 2.

In addition, referring to FIG. 4, the case in which the type of the virtual animal character to be identified is one of a plurality of types is described as an example, but an embodiment of the present disclosure is not limited thereto. For example, the type of the identified virtual animal character may be identified as including the plurality of types. For example, in case that the virtual animal character is a frog, the type of the virtual animal character may include both the aerial organism and the land organism. That is, the type of the virtual animal character for the frog may be the aerial organism and the land organism. In this case, a value of the weights according to a type of an exercise may be set as illustrated in the following table.

**[Table 3]**

| | Land exercise | Aquatic exercise | Aerial exercise |
|---|---|---|---|
| Land organism | 2 | 1.2 | 1 |
| Aquatic organism | 1.2 | 2 | 1 |

Referring to the above-described table, in case that a type of a virtual animal character is the land organism, a weight value may be set to 2 in case of the land exercise, a weight value may be set to 1.2 in case of the aquatic exercise, and a weight value may be set to 1 in case of the aerial exercise. In case that a type of a virtual animal character is an aquatic organism, a weight value may be set to 1.2 in case of the land exercise, a weight value may be set to 2 in case of the aquatic exercise, and a weight value may be set to 1 in case of the aerial exercise. According to an embodiment, a value for changing a shape of a visual object may be identified based on a maximum value from among the weights. For example, in case that a type of an exercise performed by the user is the land exercise, the value for changing the shape of the visual object may be identified based on the weight value of 2. For example, in case that a type of an exercise performed by the user is the aquatic exercise, the value for changing the shape of the visual object may be identified based on the weight value of 2. For example, in case that a type of an exercise performed by the user is the aerial exercise, the value for changing the shape of the visual object may be identified based on the weight value of 1. In other words, in case that the virtual animal character is a frog, an applicable weight value may be referred to as the following table.

**[Table 4]**

| | Land exercise | Aquatic exercise | Aerial exercise |
|---|---|---|---|
| Frog | 2 | 2 | 1 |

According to an embodiment, a shape of a visual object for a virtual animal character may be identified based on a health condition of a user. For example, the shape of the visual object for the virtual animal character may be identified based on a degree of obesity of the user. Unlike changing the shape of the visual object according to the identified second value or the third value as the user performs the exercise, the shape of the visual object may vary according to the degree of obesity of the user even with the identical second value or the identical third value. For example, in the case of a user with a high degree of obesity, a shape of a visual object may be displayed larger than a shape of a visual object of a user with a low degree of obesity. Alternatively, in the case of a user with a high degree of obesity to a specific body part, a shape of a visual object may be displayed in a state in which a part corresponding to the specific body part is visually emphasized. The health condition of the user may include indicators related to an appearance of the user, such as a user's obesity degree and user's body information.

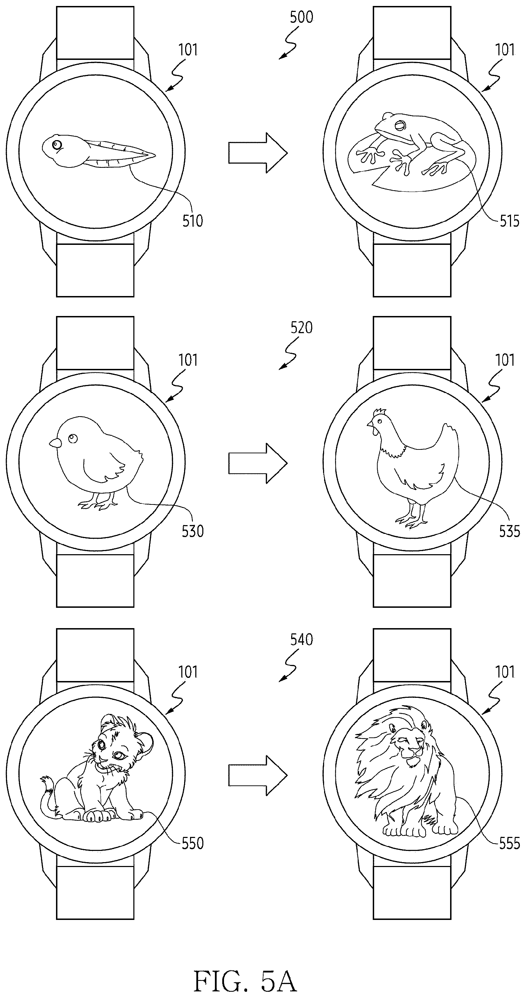

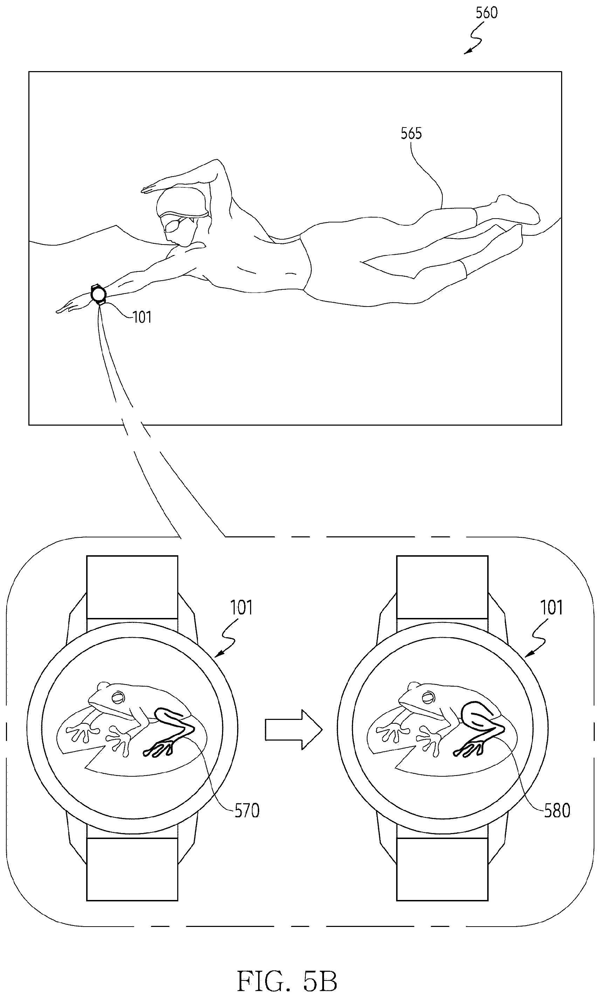

FIGS. 5A and 5B illustrate examples of changing a shape of a visual object for a virtual animal character based on data for a motion according to an embodiment of the present disclosure.

The motion may include a linear exercise and a rotational exercise. For example, a change in the motion may include a movement of a representative point displaying a wearable device (or a user) and a change (i.e., a change in an orientation) in a reference direction related to the wearable device (or the user). The visual object may indicate an object displayed via the wearable device to represent the virtual animal character. The shape may indicate a form of an exterior of the visual object.

Examples of FIGS. 5A and 5B may indicate an example of changing a shape of a virtual animal character performed based on the method of FIG. 4. Therefore, a wearable device 101 of FIGS. 5A and 5B may be understood to be substantially identical to the wearable device 101 of FIGS. 3A and 3B.

Referring to FIG. 5A, a first example 500 illustrates an example in which a virtual animal character is a frog. In the first example 500, a wearable device 101 may display a visual object 510 representing a tadpole via a display (e.g., the display module 160 of FIG. 1) of the wearable device 101. For example, the visual object 510 may be displayed by using a user interface of a second software application. Alternatively, the visual object 510 may be displayed via a user interface of another software application based on information on the visual object 510 in a state that the second software application is executed in a background. The wearable device 101 (or a processor (e.g., the processor 120 of FIG. 1)) may identify a value (e.g., the second value or the third value) for changing a shape of the visual object 510 based on data for motion obtained via a sensor while a user performs an exercise. In case that the value for changing the shape of the visual object 510 exceeds a reference value, the shape of the visual object 510 may be changed to a shape of a visual object 515. The shape of the visual object 515 may represent a frog. In case that the value for changing the shape of the visual object 510 is less than or equal to the reference value, the shape of the visual object 510 may be maintained.

A second example 520 illustrates an example in which a virtual animal character is a chicken. In the second example 520, the wearable device 101 may display a visual object 530 representing a chick via the display (e.g., the display module 160 of FIG. 1) of the wearable device 101. For example, the visual object 530 may be displayed by using the user interface of the second software application. Alternatively, the visual object 530 may be displayed via a user interface of another software application based on information on the visual object 530 in the state that the second software application is executed in the background. The wearable device 101 (or the processor (e.g., the processor 120 of FIG. 1)) may identify a value (e.g., the second value or the third value) for changing a shape of the visual object 530 based on data for a motion obtained via the sensor while the user performs an exercise. In case that the value for changing the shape of the visual object 530 exceeds the reference value, the shape of the visual object 530 may be changed to a shape of a visual object 535. The shape of the visual object 535 may represent a chicken. In case that the value for changing the shape of the visual object 530 is less than or equal to the reference value, the shape of the visual object 530 may be maintained.

A third example 540 illustrates an example in which a virtual animal character is a lion. In the third example 540, the wearable device 101 may display a visual object 550 representing a lion on the display (e.g., the display module 160 of FIG. 1) of the wearable device 101. For example, the visual object 550 may be displayed by using the user interface of the second software application. Alternatively, the visual object 550 may be displayed via a user interface of another software application based on information on the visual object 550 in the state that the second software application is executed in the background. The wearable device 101 (or the processor (e.g., the processor 120 of FIG. 1)) may identify a value (e.g., the second value or the third value) for changing a shape of the visual object 550 based on data for a motion obtained via the sensor while the user performs an exercise. In case that the value for changing the shape of the visual object 550 exceeds the reference value, the shape of the visual object 550 may be changed to a shape of a visual object 555. The shape of the visual object 555 may represent a lion. Herein, the shape of the visual object 555 may be compared with the shape of the visual object 550 to represent a grown lion. In case that the value for changing the shape of the visual object 550 is less than or equal to the reference value, the shape of the visual object 550 may be maintained.

Referring to FIG. 5B, a fourth example 560 illustrates an example in which a user 565 performs swimming in a state of wearing the wearable device 101. For example, swimming may be accompanied by a stronger load on a lower body than on a part of an upper body of the user 565. Accordingly, a type of swimming may be identified as an aquatic exercise, and a partial type of swimming may be identified as a lower body. In case that a virtual animal character of the user 565 is a frog, a visual object 570 before the user 565 swims may represent a general shape of the frog. The visual object 580 after the user 565 swims may represent a shape of a frog with a more developed lower body compared to a frog represented by the visual object 570.

Examples of FIGS. 5A and 5B illustrate that the shape of the visual object includes two shapes (e.g., a tadpole/a frog, a chick/a chicken, a baby lion/a lion), but an embodiment of the present disclosure is not limited thereto. For example, the shapes of the visual object may include four shapes, and accordingly, the reference value may include a first reference value, a second reference value, and a third reference value. The first reference value may be set lower than the second reference value, and the second reference value may be set lower than the third reference value. Although not illustrated in FIGS. 5A and 5B, according to an embodiment, the wearable device 101 (or the processor) may display a remaining value from a value (e.g., the second value or the third value) for changing the shape of the visual obj ect to one reference value of the reference values together with the visual object. The one reference value may indicate the reference value closest to a value for changing the shape of the current visual object.

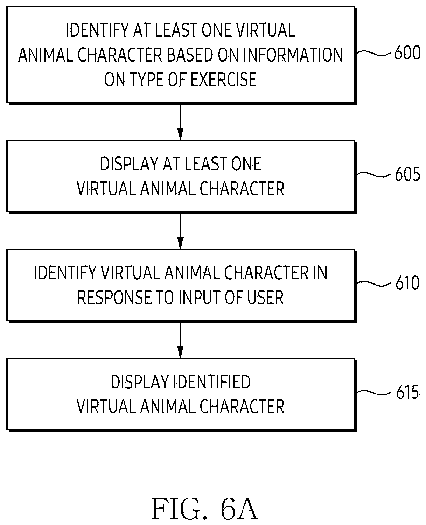

FIG. 6A is a flowchart illustrating an example of a method of proposing a virtual animal character identified based on data for a motion according to an embodiment of the present disclosure.

The motion may include a linear exercise and a rotational exercise. For example, a change in the motion may include a movement of a representative point displaying a wearable device (or a user) and a change (i.e., a change in orientation) in a reference direction related to the wearable device (or the user).

The flowchart of FIG. 6A may be performed by the wearable device 101 of FIGS. 3A and 3B. For example, the flowchart of FIG. 6A may be performed by a processor (e.g., the processor 120 of FIG. 1) of the wearable device 101.

Referring to FIG. 6A, in an operation 600, the processor may identify at least one virtual animal character based on information on a type of an exercise. For example, the processor may identify at least one virtual animal character corresponding to a type of an exercise and an exercise frequently performed by the user based on the information on the type of the exercise stored. For example, in the case of a user who frequently and regularly swims, the processor may identify aerial organisms. Alternatively, as swimming is an exercise in which a lower body is mainly used, the processor may identify an animal (e.g., a cheetah that runs fast) character with a developed lower body. Alternatively, the processor may identify a frog, which is an aquatic organism and an animal with a developed lower body.

Information on the stored type of the exercise may include not only information on a type of an exercise identified based on data for a motion of an exercise performed by the user in real-time, but also information on a type of an exercise identified based on data for a motion of an exercise previously performed by the user or a type of an exercise previously performed by the user. In other words, the information on the stored type of the exercise may include recorded information on the exercise performed by the user. According to an embodiment, the processor may store information on the exercise and the type of the exercise performed by the user in memory (e.g., the memory 130 of FIG. 1).

In an operation 605, the processor may display the identified at least one virtual animal character. For example, in the operation 600, the processor may display the at least one virtual animal character identified based on the information on the type of the exercise via a display. The processor may display a visual object corresponding to the identified at least one virtual animal character via a user interface of a second software application.

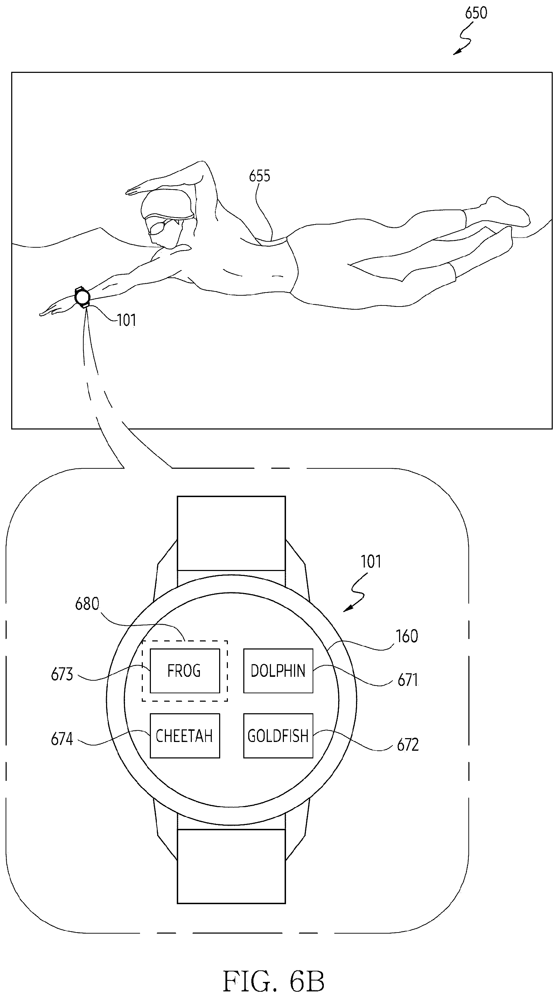

In an operation 610, the processor may identify a virtual animal character in response to an input of the user. For example, the processor may identify, in response to an input of the user to an area of a visual object displayed via the display, a virtual animal character corresponding to the visual object in the area. In case that there are four visual objects for at least one virtual animal character displayed via the display, the processor may identify one visual object and a virtual animal character corresponding to the one visual object according to an input of the user. A specific example related to this is described below in FIG. 6B.

In an operation 615, the processor may display the identified virtual animal character. For example, the processor may display the virtual animal character identified based on the input of the user via the display (e.g., the display module 160 of FIG. 1). For example, a visual object representing the identified virtual animal character may be displayed by using the user interface of the second software application. Alternatively, the visual object representing the identified virtual animal character may be displayed via a user interface of another software application based on information on the visual object in a state that the second software application is executed in a background. Alternatively, the visual object representing the identified virtual animal character may be displayed on a home screen of the wearable device in the state that the second software application is executed in the background.

According to an embodiment, the visual object representing the identified virtual animal character may be shared with the user of the wearable device and other users. For example, the information on the visual object may be provided to users of other accounts stored in an account of the user. In addition, the visual object may be uploaded to SNS of the user and provided to other users.

Referring to the above, the wearable device 101 may propose (or recommend) a virtual animal character corresponding to an exercise habit of the user based on the exercise habit of the user. Accordingly, the user may select a virtual animal character that is similar to a type and a partial type (e.g., a lower body type and an upper body type, and the like) similar to the exercise habit of the user. The user may easily grow the virtual animal character than in case that growing a virtual animal character that is not similar to the exercise habit of the user.

According to an embodiment, the processor may display a notification of the proposal to the user via a user interface of a software application (e.g., the second software application or the other software application). For example, the processor may display a pop-up notification (e.g., "In case that you select a character that matches a type of an exercise, a growth rate of a character may increase. ") for the proposal. In addition, in case that the processor may perform an exercise of a type different from the virtual animal character already selected by the user, or the user select a virtual animal character, the processor may display a notification for the above proposal via a pop-up.

A device and a method for obtaining a value for changing a shape of a virtual object (e.g., a visual object for a virtual animal character) according to an embodiment of the present disclosure may effectively motivate the user to perform an exercise and provide an active user experience by proposing a virtual animal character similar to an exercise habit of the user.

FIG. 6B illustrates an example of proposing a virtual animal character identified based on data for a motion according to an embodiment of the present disclosure.

The motion may include a linear exercise and a rotational exercise. For example, a change in the motion may include a movement of a representative point displaying the wearable device (or the user) and a change (i.e., a change in an orientation) in a reference direction related to the wearable device (or the user).

An example of FIG. 6B may indicate an example of changing a shape of a virtual animal character based on the method of FIG. 6A. Accordingly, a wearable device 101 of FIG. 6B may be understood to be substantially identical to the wearable device 101 of FIGS. 3A and 3B.

Referring to FIG. 6B, an example 650 illustrates an example in which a user 655 performs swimming in a state of wearing the wearable device 101. The example 650 only illustrates a case in which the user 655 performs swimming, but does not indicate that the user 655 performs swimming in real-time. Swimming is merely an example of an exercise performed by the user 655, and the present disclosure is not limited thereto.

According to an embodiment, the processor may identify at least one virtual animal character corresponding to an exercise and a type of an exercise frequently performed by the user based on information on a stored type of an exercise of the user 655. For example, in case that the exercise performed by the user 655 is swimming, the processor may identify at least one virtual animal character related to swimming. For example, the at least one virtual animal character may include a dolphin, a goldfish, a frog, a cheetah, and the like. The processor may identify the dolphin, the goldfish, and the frog as an aquatic organism corresponding to an aquatic exercise. In addition, the processor may identify a cheetah with developed legs, by considering that a partial type of swimming is a lower body. The frog may be identified as a virtual animal character corresponding to both an aquatic exercise and a lower body type. The at least one virtual animal character may indicate an animal proposed (or recommended) by the processor according to an exercise habit of the user 655.

According to an embodiment, the processor may display a visual object for at least one virtual animal character via a display 160. For example, the processor may display a first visual object 671 to indicate the dolphin, a second visual object 672 to indicate the goldfish, a third visual object 673 to indicate the frog, and a fourth visual object 674 to indicate the cheetah. The first visual object 671 to the fourth visual object 674 may be displayed via a user interface of a second software application. In FIG. 6B, an example of displaying a visual object indicating the at least one virtual animal character in a letter is illustrated, but the processor may display a visual object in a form of an image as illustrated in FIG. 5A.

According to an embodiment, the processor may receive an input of the user for a visual object. For example, the processor may identify a virtual animal character corresponding to a visual object in an area in response to an input of the user to the area of the visual object displayed via the display 160. For example, the processor may identify a frog as the virtual animal character in response to an input of the user to an area 680.

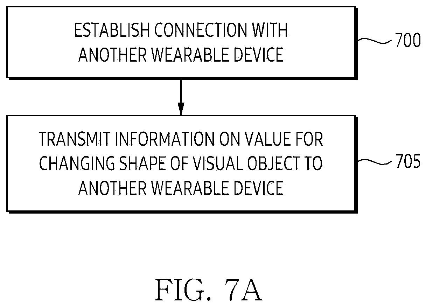

FIG. 7A is a flowchart illustrating an example of transmitting a value for changing a shape of a visual object for a virtual animal character to another wearable device according to an embodiment of the present disclosure.

The other wearable device may indicate another device different from the wearable device 101 of FIGS. 3A and 3B. For example, the other wearable device may include a head mounted display (HMD), such as AR glasses. The other wearable device may display a virtual environment and a visual object in the virtual environment via a display.

The flowchart of FIG. 7A may be performed by the wearable device 101 of FIGS. 3A and 3B. For example, the flowchart of FIG. 7A may be performed by a processor (e.g., the processor 120 of FIG. 1) of the wearable device 101.

Referring to FIG. 7A, in an operation 700, the processor may establish a connection with another wearable device. For example, the processor may perform the connection with the other wearable device worn by a user. According to an embodiment, in case that a second software application is executed, the processor may perform the connection with the other wearable device.

In an operation 705, the processor may transmit information on a value for changing a shape of a visual object to the other wearable device. The value for changing the shape of the visual object may be a value identified based on information on an exercising time, information on consumption calories, a type of an exercise and a type of a virtual animal character. For example, the value for changing the shape of the visual object may include the second value identified in the operation 415 of FIG. 4 or the third value identified in the operation 425. The processor may transmit information on the second value or the third value to the connected other wearable device.

According to an embodiment, the information on the value for changing the shape of the visual object may be used to determine another shape. For example, the information on the value to change the shape of the visual object may be used to determine the shape of the other visual object in a virtual environment displayed via a display of the other wearable device. The other visual object may be a visual object for representing the other virtual animal character. The virtual environment may include a visual object corresponding to the user of the other wearable device and a visual object for the other virtual animal character.

Referring to the above, the wearable device 101 may transmit a value for changing the shape of the visual object, which is identified according to a result of an exercise of the user, to another wearable device. The value for changing the shape of the visual object may be used to change a shape of another visual object that may be displayed via the other wearable device. The visual object may be a visual object for a virtual animal character displayed via the wearable device 101. The other visual object may be a visual object for another virtual animal character in a virtual environment that may be displayed via the other wearable device. The user of the wearable device 101 may grow a virtual object (e.g., the other virtual animal character) in the virtual environment via the result of the exercise performed by the user. A device and a method according to embodiments of the present disclosure may motivate the user to perform an exercise. The device and the method according to embodiments of the present disclosure may provide the user with a continuous user experience in which an exercise in a real environment is linked to the virtual environment.

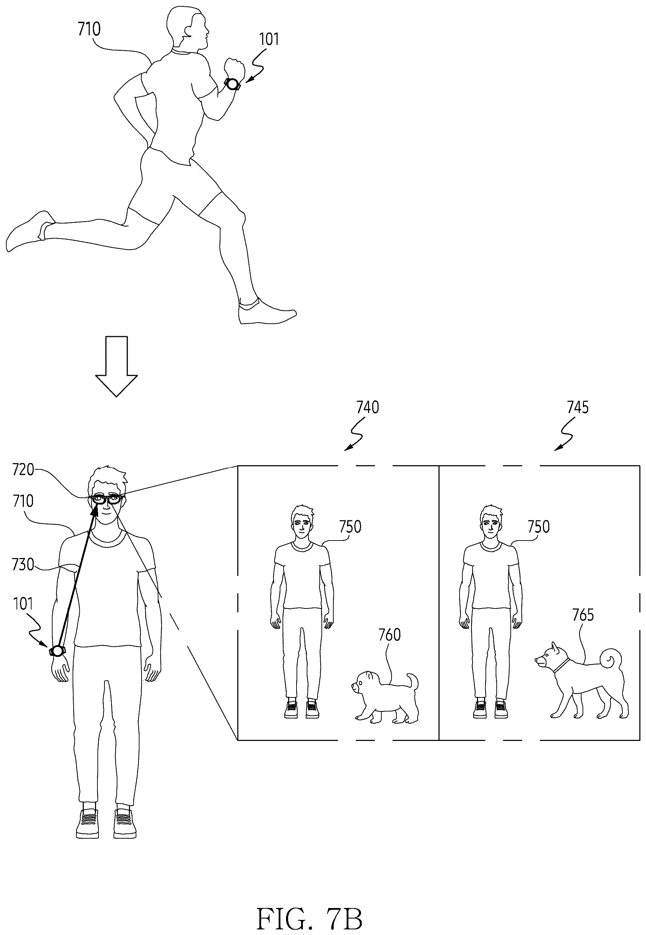

FIG. 7B illustrates an example of changing a shape of a virtual object displayed via another wearable device based on a value for changing a shape of a visual object for a virtual animal character according to an embodiment of the present disclosure.

The other wearable device may indicate another device different from the wearable device 101 of FIGS. 3A and 3B. For example, the other wearable device may include a head mounted display (HMD), such as AR glasses. The other wearable device may display a virtual environment and a visual object in the virtual environment via the display. The virtual object may indicate a visual object for another virtual animal character different from a virtual animal character displayed via the wearable device 101.

An example of FIG. 7B may indicate an example in which a shape of the other virtual animal character is changed based on the method of FIG. 7A. Therefore, a wearable device 101 of FIG. 7B may be understood to be substantially identical to the wearable device 101 of FIGS. 3A and 3B. Another wearable device 720 of FIG. 7B may include AR glasses.

Referring to FIG. 7B, a user 710 may perform an exercise in a state of wearing the wearable device 101. For example, the user 710 may perform running in the state of wearing the wearable device 101. According to an embodiment, the user 710 may transmit information 730 on a value for changing a shape of a visual object to the other wearable device 720 by the wearable device 101. For example, the information 730 on the value for changing the shape of the visual object may indicate information on time when the user 710 performed the running, information on consumption calories by the running, and a weight value identified according to whether a type of an exercise (e.g., a land exercise) corresponds to a type of virtual animal character. The wearable device 101 may be in a state in which connection with another wearable device 720 has been established. The type of the virtual animal character may be a type of another virtual animal character of the other wearable device 720 other than the virtual animal character of the wearable device 101.

A first example 740 and a second example 745 illustrate an example of displaying visual objects in a virtual environment via the other wearable device 720. The first example 740 and the second example 745 illustrate an example in which the user 710 views the virtual environment from a third-person perspective. The first example 740 may indicate a situation before the information 730 is received, and the second example 745 may indicate a situation in which the shape of the visual object is changed based on the information 730 after the information 730 is received. Referring to the first example 740, the other wearable device 720 may display a visual object 750 corresponding to the user 710 and a visual object 760 for the other virtual animal character via a display. For example, the other virtual animal character may be a puppy. Referring to the second example 745, the other wearable device 720 may change a shape of the visual object 760 into a shape of the visual object 765 based on the received information 730 and display it. For example, the visual object 765 may indicate a grown dog, which a visual object 760 indicating the puppy has grown. As described above, the user 710 of the wearable device 101 may grow a virtual object (e.g., the visual object 760) in the virtual environment via a result of an exercise performed by the user 710.

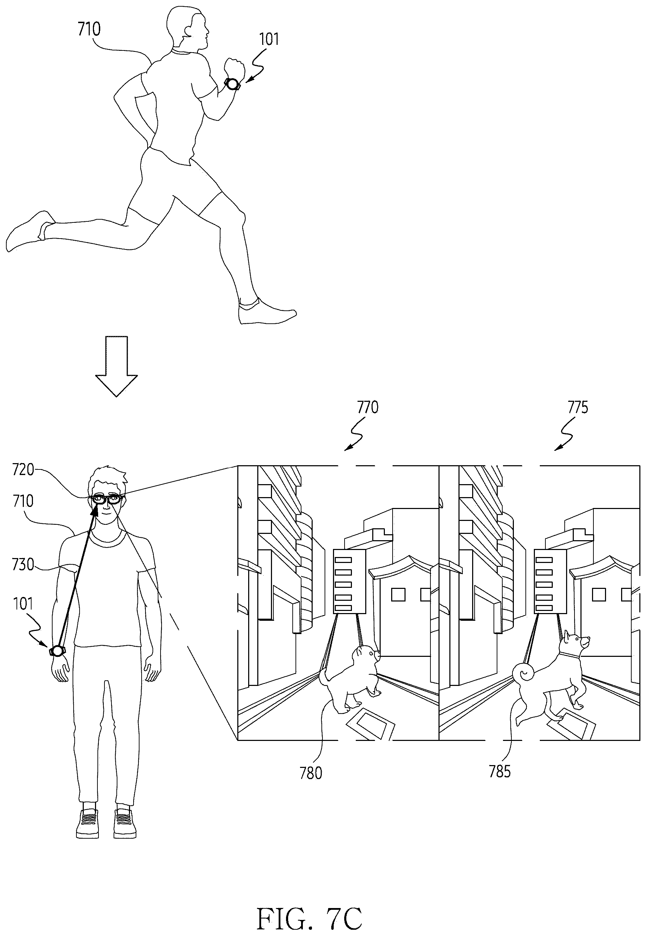

FIG. 7C illustrates another example of changing a shape of a virtual object displayed via another wearable device based on a value for changing a shape of a visual object for the virtual animal character according to an embodiment of the present disclosure.

The other wearable device may indicate another device different from the wearable device 101 of FIGS. 3A and 3B. For example, the other wearable device may include a head mounted display (HMD), such as AR glasses. The other wearable device may display a virtual environment and a visual object in the virtual environment via the display. The virtual object may indicate a visual object for a virtual animal character different from the virtual animal character displayed via the wearable device 101.

An example of FIG. 7C may indicate an example in which a shape of the other virtual animal character is changed based on the method of FIG. 7A. Therefore, a wearable device 101 of FIG. 7C may be understood to be substantially identical to the wearable device 101 of FIGS. 3A and 3B. The other wearable device 720 of FIG. 7B may include AR glasses.

Referring to FIG. 7C, a user 710 may perform an exercise in a state of wearing the wearable device 101. For example, the user 710 may perform running in the state of wearing the wearable device 101. According to an embodiment, the user 710 may transmit information 730 on a value for changing a shape of a visual object to the other wearable device 720 by the wearable device 101. For example, the information 730 on the value for changing the shape of the visual object may indicate information on the time when the user 710 performed the running, information on consumption calories by the running, and a weight value identified according to whether a type of an exercise (e.g., a land exercise) corresponds to a type of a virtual animal character. The wearable device 101 may be in a state in which connection with the other wearable device 720 has been established. The type of the virtual animal character may be a type of another virtual animal character of the other wearable device 720 other than the virtual animal character of the wearable device 101.

A third example 770 and a fourth example 775 illustrate an example of displaying visual objects in a virtual environment via the other wearable device 720. The third example 770 and the fourth example 775 illustrate an example in which the user 710 views the virtual environment from a first-person perspective. The third example 770 may indicate a situation before the information 730 is received, and the fourth example 775 may indicate a situation in which a shape of the visual object is changed based on the information 730 after the information 730 is received. Referring to the third example 770, the other wearable device 720 may display a visual object 780 for the other virtual animal character via a display. For example, the other virtual animal character may be a puppy. Referring to the fourth example 775, the other wearable device 720 may change a shape of the visual object 780 into a shape of a visual object 785 based on the received information 730 and display it. For example, the visual object 785 may indicate a dog in which the visual object 780 indicating the puppy has grown. As described above, the user 710 of the wearable device 101 may grow a virtual object (e.g., the visual object 780) in the virtual environment via a result of an exercise performed by the user 710.

As described above, a wearable device 101 may comprise a sensor 176. The wearable device 101 may comprise a display 160. The wearable device 101 may comprise a processor 120. The processor 120 may be configured to obtain data for a motion of the wearable device 101 via the sensor 176 by using a first software application for health. The processor 120 may be configured to, based on the data, identify a type of an exercise performed by a user wearing the wearable device 101 while the data is obtained by using the first software application. The processor 120 may be configured to identify whether the type of the exercise corresponds to a type of a virtual animal character by using a second software application for displaying a visual object for a virtual animal character. The processor 120 may be configured to, in response to the type of the exercise being different from the type of the virtual animal character, change a first value stored for changing a shape of the virtual object to a second value higher than the first value and identified based on the data, and identify a shape of the virtual object based on the second value. The processor 120 may be configured to, in response to the type of the exercise being corresponding to the type of the virtual animal character, change the first value to a third value higher than the second value and identified based on the data, and identify a shape of the virtual object based on the third value. The processor 120 may be configured to, in response to a predetermined event, display via the display 160 the visual object having the identified shape. The second value and the third value may be identified based on information on exercising time of the exercise and information on consumption calories according to the exercise identified from the data.

According to an embodiment, the processor 120 may be configured to, in response to the type of the exercise being different from the type of the virtual animal character, set the shape of the virtual object as a second shape changed from a first shape based on the second value higher than a reference value, and maintain the shape as the first shape based on the second value lower than or equal to the reference value. The processor 120 may be configured to, in response to the type of the exercise being corresponding to the type of the virtual animal character, set the shape of the virtual object as a second shape changed from a first shape based on the third value higher than a reference value, and maintain the shape as the first shape based on the third value lower than or equal to the reference value.

According to an embodiment, the processor 120 may be configured to identify a partial type for the type of the exercise based on the data. The processor 120 may be configured to, in response to the type of the exercise being different from the type of the virtual animal character, based on the second value lower than or equal to the reference value and the identified partial type, set a third shape changed from the first shape of the visual object. The third shape may represent a shape in which a portion of the first shape corresponding to the partial type is changed. The partial type may include an upper body exercise type and a lower body exercise type.

According to an embodiment, the processor 120 may be configured to identify at least one virtual animal character corresponding to the type of the exercise based on the information on the type of the identified exercise. The processor 120 may be configured to display via the display 160 information on the at least one virtual animal character by using a user interface of the second software application.

According to an embodiment, the sensor 176 may include at least one of a global positioning system (GPS), a gyro sensor, a geomagnetic sensor, a photoplethysmogram sensor, an electrocardiogram sensor, or a bioelectrical impedance analysis sensor.

According to an embodiment, the processor 120 may be configured to identify a first partial value for any unit time based on the information on the exercising time. The processor 120 may be configured to identify a second partial value for any unit consumption calories based on the information on the consumption calories. The processor 120 may be configured to identify each of the second value and the third value based on the first partial value and the second partial value.

According to an embodiment, the type of the virtual animal character may include at least one of land, aqua, or air. The type of the exercise may include at least one of land exercise, aquatic exercise, or aerial exercise.

According to an embodiment, in case that the type of the virtual animal character includes the land and the aqua, the third value may be identified based on a maximum value from among a first weight value for a case that the type of the virtual animal character is the land and a second weight vale for a case that the type of the virtual animal character is the aqua. Each of the first weight value and the second weight value may be identified according to the type of the exercise.

According to an embodiment, in case that the type of the exercise includes the land exercise and the aquatic exercise, the third value may be identified based on a maximum value from among a third weight value for a case that the type of the exercise is the land exercise and a fourth weight vale for a case that the type of the exercise is the aquatic exercise. Each of the third weight value and the fourth weight value may be identified according to the type of the virtual animal character.

According to an embodiment, the processor 120 may be configured to transmit, to another wearable device 101, connected to the wearable device 101, worn by the user, the second value or the third value for the virtual animal character. The second value and the third value may be used to determine a shape of another visual object in a virtual environment displayed via the display 160 of the another wearable device 101. The another visual object may be a visual object for representing another virtual animal character.

As described above, a method performed by a wearable device 101 may comprise obtaining data for a motion of the wearable device 101 via a sensor 176 of the wearable device 101 by using a first software application for health. The method may comprise, based on the data, identifying a type of an exercise performed by a user wearing the wearable device 101 while the data is obtained by using the first software application. The method may comprise identifying whether the type of the exercise corresponds to a type of a virtual animal character by using a second software application for displaying a visual object for a virtual animal character. The method may comprise, in response to the type of the exercise being different from the type of the virtual animal character, changing a first value stored for changing a shape of the virtual object to a second value higher than the first value and identified based on the data, and identifying a shape of the virtual object based on the second value. The method may comprise, in response to the type of the exercise being corresponding to the type of the virtual animal character, changing the first value to a third value higher than the second value and identified based on the data, and identifying a shape of the virtual object based on the third value. The method may comprise, in response to a predetermined event, displaying via a display 160 of the wearable device 101 the visual object having the identified shape. The second value and the third value are identified based on information on exercising time of the exercise and information on consumption calories according to the exercise identified from the data.

According to an embodiment, the method may comprise, in response to the type of the exercise being different from the type of the virtual animal character, setting the shape of the virtual object as a second shape changed from a first shape based on the second value higher than a reference value, and maintaining the shape as the first shape based on the second value lower than or equal to the reference value. The method may comprise, in response to the type of the exercise being corresponding to the type of the virtual animal character, setting the shape of the virtual object as a second shape changed from a first shape based on the third value higher than a reference value, and maintaining the shape as the first shape based on the third value lower than or equal to the reference value.

According to an embodiment, the method may comprise identifying a partial type for the type of the exercise based on the data. The method may comprise, in response to the type of the exercise being different from the type of the virtual animal character, based on the second value lower than or equal to the reference value and the identified partial type, setting a third shape changed from the first shape of the visual object. The third shape may represent a shape in which a portion of the first shape corresponding to the partial type is changed. The partial type may include an upper body exercise type and a lower body exercise type.

According to an embodiment, the method may comprise identifying at least one virtual animal character corresponding to the type of the exercise based on the information on the type of the identified exercise. The method may comprise displaying via the display 160 information on the at least one virtual animal character by using a user interface of the second software application.

According to an embodiment, the sensor 176 may include at least one of a global positioning system (GPS), a gyro sensor, a geomagnetic sensor, a photoplethysmogram sensor, an electrocardiogram sensor, or a bioelectrical impedance analysis sensor.

According to an embodiment, the method may comprise identifying a first partial value for any unit time based on the information on the exercising time. The method may comprise identifying a second partial value for any unit consumption calories based on the information on the consumption calories. The method may comprise identifying each of the second value and the third value based on the first partial value and the second partial value.

According to an embodiment, the type of the virtual animal character may include at least one of land, aqua, or air. The type of the exercise may include at least one of land exercise, aquatic exercise, or aerial exercise.

According to an embodiment, in case that the type of the virtual animal character includes the land and the aqua, the third value may be identified based on a maximum value from among a first weight value for a case that the type of the virtual animal character is the land and a second weight vale for a case that the type of the virtual animal character is the aqua. Each of the first weight value and the second weight value may be identified according to the type of the exercise.

According to an embodiment, in case that the type of the exercise includes the land exercise and the aquatic exercise, the third value may be identified based on a maximum value from among a third weight value for a case that the type of the exercise is the land exercise and a fourth weight vale for a case that the type of the exercise is the aquatic exercise. Each of the third weight value and the fourth weight value may be identified according to the type of the virtual animal character.

According to an embodiment, the method may comprise transmitting, to another wearable device 101, connected to the wearable device 101, worn by the user, the second value or the third value for the virtual animal character. The second value and the third value may be used to determine a shape of another visual object in a virtual environment displayed via the display 160 of the another wearable device 101. The another visual object may be a visual object for representing another virtual animal character.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," or "connected with" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between a case in which data is semi-permanently stored in the storage medium and a case in which the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable device (101) comprising:
a sensor (176);
a display (160); and
a processor (120),
wherein the processor (120) is configured to:
obtain data for a motion of the wearable device (101) via the sensor (176) by using a first software application for health,
based on the data, identify a type of an exercise performed by a user wearing the wearable device (101) while the data is obtained by using the first software application,
identify whether the type of the exercise corresponds to a type of a virtual animal character by using a second software application for displaying a visual object for a virtual animal character,
in response to the type of the exercise being different from the type of the virtual animal character, change a first value stored for changing a shape of the virtual object to a second value higher than the first value and identified based on the data, and identify a shape of the virtual object based on the second value,
in response to the type of the exercise being corresponding to the type of the virtual animal character, change the first value to a third value higher than the second value and identified based on the data, and identify a shape of the virtual object based on the third value, and
in response to a predetermined event, display via the display (160) the visual object having the identified shape,
wherein the second value and the third value are identified based on information on exercising time of the exercise and information on consumption calories according to the exercise identified from the data.

2. The wearable device (101) of claim 1,
wherein the processor (120) is configured to:
in response to the type of the exercise being different from the type of the virtual animal character, set the shape of the virtual object as a second shape changed from a first shape based on the second value higher than a reference value, and maintain the shape as the first shape based on the second value lower than or equal to the reference value, and
in response to the type of the exercise being corresponding to the type of the virtual animal character, set the shape of the virtual object as a second shape changed from a first shape based on the third value higher than a reference value, and maintain the shape as the first shape based on the third value lower than or equal to the reference value.

3. The wearable device (101) of claim 2,
wherein the processor (120) is configured to:
identify a partial type for the type of the exercise based on the data, and
in response to the type of the exercise being different from the type of the virtual animal character, based on the second value lower than or equal to the reference value and the identified partial type, set a third shape changed from the first shape of the visual object,
wherein the third shape represents a shape in which a portion of the first shape corresponding to the partial type is changed, and
wherein the partial type includes an upper body exercise type and a lower body exercise type.

4. The wearable device (101) of claim 1,
wherein the processor (120) is configured to:
identify at least one virtual animal character corresponding to the type of the exercise based on the information on the type of the identified exercise, and
display via the display (160) information on the at least one virtual animal character by using a user interface of the second software application.

5. The wearable device (101) of claim 1,
wherein the sensor (176) includes at least one of a global positioning system (GPS), a gyro sensor, a geomagnetic sensor, a photoplethysmogram sensor, an electrocardiogram sensor, or a bioelectrical impedance analysis sensor.

6. The wearable device (101) of claim 1,
wherein the processor (120) is configured to:
identify a first partial value for any unit time based on the information on exercising time,
identify a second partial value for any unit consumption calories based on the information on consumption calories, and
identify each of the second value and the third value based on the first partial value and the second partial value.

7. The wearable device (101) of claim 1,
wherein the type of the virtual animal character includes at least one of land, aqua, or air, and
wherein the type of the exercise includes at least one of land exercise, aquatic exercise, or aerial exercise.

8. The wearable device (101) of claim 7,
wherein, in case that the type of the virtual animal character includes the land and the aqua, the third value is identified based on a maximum value from among a first weight value for a case that the type of the virtual animal character is the land and a second weight vale for a case that the type of the virtual animal character is the aqua, and
wherein each of the first weight value and the second weight value is identified according to the type of the exercise.

9. The wearable device (101) of claim 7,
wherein, in case that the type of the exercise includes the land exercise and the aquatic exercise, the third value is identified based on a maximum value from among a third weight value for a case that the type of the exercise is the land exercise and a fourth weight vale for a case that the type of the exercise is the aquatic exercise, and
wherein each of the third weight value and the fourth weight value is identified according to the type of the virtual animal character.

10. The wearable device (101) of claim 1,
wherein the processor (120) is configured to:
transmit, to another wearable device, connected to the wearable device (101), worn by the user, the second value or the third value for the virtual animal character,
wherein the second value and the third value are used to determine a shape of another visual object in a virtual environment displayed via the display (160) of the another wearable device, and
wherein the another visual object is a visual object for representing another virtual animal character.

11. A method performed by a wearable device (101) comprising:
obtaining data for a motion of the wearable device (101) via a sensor (176) of the wearable device (101) by using a first software application for health,
based on the data, identifying a type of an exercise performed by a user wearing the wearable device (101) while the data is obtained by using the first software application,
identifying whether the type of the exercise corresponds to a type of a virtual animal character by using a second software application for displaying a visual object for a virtual animal character,
in response to the type of the exercise being different from the type of the virtual animal character, changing a first value stored for changing a shape of the virtual object to a second value higher than the first value and identified based on the data, and identifying a shape of the virtual object based on the second value,
in response to the type of the exercise being corresponding to the type of the virtual animal character, changing the first value to a third value higher than the second value and identified based on the data, and identifying a shape of the virtual object based on the third value, and
in response to a predetermined event, displaying via a display of the wearable device (101) the visual object having the identified shape,
wherein the second value and the third value are identified based on information on exercising time of the exercise and information on consumption calories according to the exercise identified from the data.

12. The method of claim 11, the method comprising:
in response to the type of the exercise being different from the type of the virtual animal character, setting the shape of the virtual object as a second shape changed from a first shape based on the second value higher than a reference value, and maintaining the shape as the first shape based on the second value lower than or equal to the reference value, and
in response to the type of the exercise being corresponding to the type of the virtual animal character, setting the shape of the virtual object as a second shape changed from a first shape based on the third value higher than a reference value, and maintaining the shape as the first shape based on the third value lower than or equal to the reference value.

13. The method of claim 12, the method comprising:
identifying a partial type for the type of the exercise based on the data, and
in response to the type of the exercise being different from the type of the virtual animal character, based on the second value lower than or equal to the reference value and the identified partial type, setting a third shape changed from the first shape of the visual object,
wherein the third shape represents a shape in which a portion of the first shape corresponding to the partial type is changed, and
wherein the partial type includes an upper body exercise type and a lower body exercise type.

14. The method of claim 11, the method comprising:
identifying at least one virtual animal character corresponding to the type of the exercise based on the information on the type of the identified exercise,
displaying via the display (160) information on the at least one virtual animal character by using a user interface of the second software application.

15. A non-transitory computer readable storage medium, when executed by a processor (120) of a wearable device (101) comprising a sensor (176) and a display, storing one or more programs including instructions that cause to:
obtain data for a motion of the wearable device (101) via the sensor (176) by using a first software application for health,
based on the data, identify a type of an exercise performed by a user wearing the wearable device (101) while the data is obtained by using the first software application,
identify whether the type of the exercise corresponds to a type of a virtual animal character by using a second software application for displaying a visual object for a virtual animal character,
in response to the type of the exercise being different from the type of the virtual animal character, change a first value stored for changing a shape of the virtual object to a second value higher than the first value and identified based on the data, and identify a shape of the virtual object based on the second value,
in response to the type of the exercise being corresponding to the type of the virtual animal character, change the first value to a third value higher than the second value and identified based on the data, and identify a shape of the virtual object based on the third value, and
in response to a predetermined event, display via the display (160) the visual object having the identified shape,
wherein the second value and the third value are identified based on information on exercising time of the exercise and information on consumption calories according to the exercise identified from the data.
